# EUROPEAN PATENT SPECIFICATION

(11) **EP 2 384 761 B1**
(45) Date of publication and mention of the grant of the patent: **04.09.2013**
(21) Application number: 10004897.4
(22) Date of filing: 07.05.2010
(51) Int. Cl.: A61K 35/76, C12N 7/00

(54) **Modified rodent parvovirus capable of propagating and spreading through human gliomas**
Modifiziertes Nagetier-Parvovirus, das sich durch menschliche Glioma vermehren und ausbreiten kann
Parvovirus de rongeurs modifié capable de se propager et se répandre par des gliomes humains

(43) Date of publication of application: 09.11.2011
(73) Proprietor: Deutsches Krebsforschungszentrum, 69120 Heidelberg (DE)
(72) Inventor: Nüesch, Jürg, 69124 Heidelberg (DE); Thomas, Nadja, 67117 Limburgerhof (DE); Plotzky, Claudia, 68549 Ilvesheim (DE); Rommelaere, Jean, 69118 Heidelberg (DE)
(74) Representative: Schüssler, Andrea

(56) References cited:
- EP-A1- 1 077 260
- DE-A1-102004 020 762
- US-A1- 2004 220 124
- US-A1- 2006 051 333
- US-A1- 2009 215 879
- HERRERO Y CALLE MARTA ET AL: "Parvovirus H-1 infection of human glioma cells leads to complete viral replication and efficient cell killing.", INTERNATIONAL JOURNAL OF CANCER, vol. 109, no. 1, 10 March 2004 (2004-03-10), pages 76-84, XP002614412, ISSN: 0020-7136
- BÄR SÉVERINE ET AL: "Vesicular egress of non-enveloped lytic parvoviruses depends on gelsolin functioning.", PLOS PATHOGENS 2008 LNKD- PUBMED:18704167, vol. 4, no. 8, 2008, page E1000126, XP002614413, ISSN: 1553-7374
- BRANDENBURGER A ET AL: "NS-1 and NS-2 proteins may act synergistically in the cytopathogenicity of parvovirus MVMp", VIROLOGY, ACADEMIC PRESS,ORLANDO, US, vol. 174, no. 2, 1 February 1990 (1990-02-01), pages 576-584, XP023050288, ISSN: 0042-6822, DOI: DOI:10.1016/0042-6822(90)90110-D [retrieved on 1990-02-01]
- NAKASHIMA A ET AL: "Human Parvovirus B19 nonstructural protein transactivates the p21/WAF1 through Sp1", VIROLOGY, ACADEMIC PRESS,ORLANDO, US, vol. 329, no. 2, 24 November 2004 (2004-11-24), pages 493-504, XP004613877, ISSN: 0042-6822
- LI X ET AL: "MUTATION OF LYSINE 405 TO SERINE IN THE PARVOVIRUS H-1 NS1 ABOLISHES ITS FUNCTIONS FOR VIRAL DNA REPLICATION, LATE PROMOTER TRANS ACTIVATION, AND CYTOTOXICITY", JOURNAL OF VIROLOGY, THE AMERICAN SOCIETY FOR MICROBIOLOGY, US, vol. 64, no. 10, 1 October 1990 (1990-10-01), pages 4654-4660, XP000867496, ISSN: 0022-538X
- RUSSELL S J ET AL: "TRANSFORMATION-DEPENDENT EXPRESSION OF INTERLEUKIN GENES DELIVERED BY A RECOMBINANT PARVOVIRUS", JOURNAL OF VIROLOGY, THE AMERICAN SOCIETY FOR MICROBIOLOGY, US, vol. 66, no. 5, 1 May 1992 (1992-05-01), pages 2821-2828, XP001000872, ISSN: 0022-538X
- KANNO HIROYUKI ET AL: "Aleutian mink disease parvovirus infection of mink peritoneal macrophages and human macrophage cell lines", JOURNAL OF VIROLOGY, vol. 67, no. 4, 1993, pages 2075-2082, XP002637367, ISSN: 0022-538X
- WOLLMANN G ET AL: "Targeting Human Glioblastoma Cells: Comparison of Nice Viruses with Oncolytic Potential", JOURNAL OF VIROLOGY, THE AMERICAN SOCIETY FOR MICROBIOLOGY, US, vol. 79, no. 10, 1 May 2005 (2005-05-01), pages 6005-6022, XP003000393, ISSN: 0022-538X, DOI: DOI:10.1128/JVI.79.10.6005-6022.2005

### SEQUENCE LISTING

<110> Deutsches Krebsforschungszentrum
<120> Modified rodent parvovirus capable of propagating and spreading through human gliomas
<130> K3357EP
<160> 12
<170> PatentIn version 3.3
<210> 1
   <211> 672
   <212> PRT
   <213> artificial sequence
<220>
   <223> NS1
<400> 1
   Met Ala Gly Asn Ala Tyr Ser Asp Glu Val Leu Gly Val Thr Asn Trp
   1 5 10 15
   Leu Lys Asp Lys Ser Ser Gln Glu Val Phe Ser Phe Val Phe Lys Asn
   20 25 30
   Glu Asn Val Gln Leu Asn Gly Lys Asp Ile Gly Trp Asn Ser Tyr Arg
   35 40 45
   Lys Glu Leu Gln Asp Asp Glu Leu Lys Ser Leu Gln Arg Gly Ala Glu
   50 55 60
   Thr Thr Trp Asp Gln Ser Glu Asp Met Glu Trp Glu Ser Ala Val Asp
   65 70 75 80
   Asp Met Thr Lys Lys Gln Val Phe Ile Phe Asp Ser Leu Val Lys Lys
   85 90 95
   Cys Leu Phe Glu Val Leu Ser Thr Lys Asn Ile Ala Pro Ser Asn Val
   100 105 110
   Thr Trp Phe Val Gln His Glu Trp Gly Lys Asp Pro Gly Trp His Cys
   115 120 125
   His Val Leu Ile Gly Gly Lys Asp Phe Ser Gln Pro Gln Gly Lys Trp
   130 135 140
   Trp Arg Arg Gln Leu Asn Val Tyr Trp Ser Arg Trp Leu Val Thr Ala
   145 150 155 160
   Cys Asn Val Gln Leu Thr Pro Ala Glu Arg Ile Lys Leu Arg Glu Ile
   165 170 175
   Ala Glu Asp Ser Glu Trp Val Thr Leu Leu Thr Tyr Lys His Lys His
   180 185 190
   Thr Lys Lys Asp Tyr Thr Lys Cys Val Leu Phe Gly Asn Met Ile Ala
   195 200 205
   Tyr Tyr Phe Leu Ser Lys Lys Lys Ile Cys Thr Ser Pro Pro Arg Asp
   210 215 220
   Gly Gly Tyr Phe Leu Ser Ser Asp Ser Gly Trp Lys Thr Asn Phe Leu
   225 230 235 240
   Lys Glu Gly Glu Arg His Leu Val Ser Lys Leu Tyr Thr Asp Glu Met
   245 250 255
   Lys Pro Glu Thr Val Glu Thr Thr Val Thr Thr Ala Gln Glu Ala Lys
   260 265 270
   Arg Gly Arg Ile Gln Thr Arg Glu Glu Val Ser Ile Lys Thr Thr Leu
   275 280 285
   Lys Glu Leu Val His Lys Arg Val Thr Ser Pro Glu Asp Trp Met Met
   290 295 300
   Met Gln Pro Asp Ser Tyr Ile Glu Met Met Ala Gln Pro Gly Gly Glu
   305 310 315 320
   Asn Leu Leu Lys Asn Thr Leu Glu Ile Cys Thr Leu Thr Leu Ala Arg
   325 330 335
   Thr Lys Thr Ala Phe Asp Leu Ile Leu Glu Lys Ala Glu Thr Ser Lys
   340 345 350
   Leu Ala Asn Phe Ser Met Ala Ser Thr Arg Thr Cys Arg Ile Phe Ala
   355 360 365
   Glu His Gly Trp Asn Tyr Ile Lys Val Cys His Ala Ile Cys Cys Val
   370 375 380
   Leu Asn Arg Gln Gly Gly Lys Arg Asn Thr Val Leu Phe His Gly Pro
   385 390 395 400
   Ala Ser Thr Gly Lys Ser Ile Ile Ala Gln Ala Ile Ala Gln Ala Val
   405 410 415
   Gly Asn Val Gly Cys Tyr Asn Ala Ala Asn Val Asn Phe Pro Phe Asn
   420 425 430
   Asp Cys Thr Asn Lys Asn Leu Ile Trp Val Glu Glu Ala Gly Asn Phe
   435 440 445
   Gly Gln Gln Val Asn Gln Phe Lys Ala Ile Cys Ser Gly Gln Thr Ile
   450 455 460
   Arg Ile Asp Gln Lys Gly Lys Gly Ser Lys Gln Ile Glu Pro Thr Pro
   465 470 475 480
   Val Ile Met Thr Thr Asn Glu Asn Ile Thr Val Val Arg Ile Gly Cys
   485 490 495
   Glu Glu Arg Pro Glu His Thr Gln Pro Ile Arg Asp Arg Met Leu Asn
   500 505 510
   Ile His Leu Thr Arg Thr Leu Pro Gly Asp Phe Gly Leu Val Asp Lys
   515 520 525
   His Glu Trp Pro Leu Ile Cys Ala Trp Leu Val Lys Asn Gly Tyr Gln
   530 535 540
   Ser Thr Met Ala Cys Tyr Cys Ala Lys Trp Gly Lys Val Pro Asp Trp
   545 550 555 560
   Ser Glu Asp Trp Ala Glu Pro Lys Leu Asp Thr Pro Ile Asn Ser Leu
   565 570 575
   Gly Ser Met Arg Ser Pro Ser Leu Thr Pro Arg Ser Thr Pro Leu Ser
   580 585 590
   Gln Asn Tyr Ala Leu Thr Pro Leu Ala Ser Asp Leu Ala Asp Leu Ala
   595 600 605
   Leu Glu Pro Trp Ser Thr Pro Asn Thr Pro Val Ala Gly Thr Ala Ala
   610 615 620
   Ser Gln Asn Thr Gly Glu Ala Gly Ser Thr Ala Cys Gln Gly Ala Gln
   625 630 635 640
   Arg Ser Pro Thr Trp Ser Glu Ile Glu Ala Asp Leu Arg Ala Cys Phe
   645 650 655
   Ser Gln Glu Gln Leu Glu Ser Asp Phe Asn Glu Glu Leu Thr Leu Asp
   660 665 670
<210> 2
   <211> 199
   <212> PRT
   <213> artificial sequence
<220>
   <223> NS2
<400> 2
   Met Ala Gly Asn Ala Tyr Ser Asp Glu Val Leu Gly Val Thr Asn Trp
   1 5 10 15
   Leu Lys Asp Lys Ser Ser Gln Glu Val Phe Ser Phe Val Phe Lys Asn
   20 25 30
   Glu Asn Val Gln Leu Asn Gly Lys Asp Ile Gly Trp Asn Ser Tyr Arg
   35 40 45
   Lys Glu Leu Gln Asp Asp Glu Leu Lys Ser Leu Gln Arg Gly Ala Glu
   50 55 60
   Thr Thr Trp Asp Gln Ser Glu Asp Met Glu Trp Glu Ser Ala Val Asp
   65 70 75 80
   Met Thr Lys Lys Phe Asn Ala Leu Thr Ile Ser Asp Ser Glu Lys Tyr
   85 90 95
   Ala Ser Gln Pro Lys Leu Arg Ser Tyr Ser Thr Cys Ile Gly Pro Cys
   100 105 110
   Gly Pro Ser Ser Arg Ala Leu Glu His Thr Lys Tyr Ser Cys Cys Gly
   115 120 125
   His Cys Ser Lys Pro Lys His Trp Gly Gly Trp Phe His Ser Leu Pro
   130 135 140
   Arg Cys Ser Thr Glu Pro Asn Leu Val Arg Asp Arg Gly Gly Phe Glu
   145 150 155 160
   Ser Leu Leu Gln Ser Arg Thr Val Gly Glu Arg Leu Gln Arg Gly Ala
   165 170 175
   Asp Leu Gly Leu Arg Tyr Gly Val Leu Met Tyr Asp Tyr Leu Phe Tyr
   180 185 190
   Arg Pro Glu Ile Thr Trp Phe
   195
<210> 3
   <211> 738
   <212> PRT
   <213> artificial sequence
<220>
   <223> VP1/2
<400> 3
   Met Ala Pro Pro Ala Lys Arg Ala Lys Arg Gly Trp Val Pro Pro Gly
   1 5 10 15
   Tyr Lys Tyr Leu Gly Pro Gly Asn Ser Leu Asp Gln Gly Glu Pro Thr
   20 25 30
   Asn Pro Ser Asp Ala Ala Ala Lys Glu His Asp Glu Ala Tyr Asp Gln
   35 40 45
   Tyr Ile Lys Ser Gly Lys Asn Pro Tyr Leu Tyr Phe Ser Pro Ala Asp
   50 55 60
   Gln Arg Phe Ile Asp Gln Thr Lys Asp Ala Lys Asp Trp Gly Gly Lys
   65 70 75 80
   Val Gly His Tyr Phe Phe Arg Thr Lys Arg Ala Phe Ala Pro Lys Leu
   85 90 95
   Ser Thr Asp Ser Glu Pro Gly Thr Ser Gly Thr Ser Gly Val Ser Arg
   100 105 110
   Pro Gly Lys Arg Thr Lys Pro Pro Ala His Ile Phe Val Asn Gln Ala
   115 120 125
   Arg Ala Lys Lys Lys Arg Ala Ser Leu Ala Ala Gln Gln Arg Thr Leu
   130 135 140
   Thr Met Ser Asp Gly Thr Glu Thr Asn Gln Pro Asp Thr Gly Ile Ala
   145 150 155 160
   Asn Ala Arg Val Glu Arg Ser Ala Asp Gly Gly Gly Ser Ser Gly Gly
   165 170 175
   Gly Gly Gly Ser Gly Gly Gly Gly Ile Gly Val Ser Thr Gly Thr Tyr
   180 185 190
   Asp Asn Gln Thr Thr Tyr Lys Phe Leu Gly Asp Gly Trp Val Glu Ile
   195 200 205
   Thr Ala His Ala Ser Arg Leu Leu His Leu Gly Met Pro Pro Ser Glu
   210 215 220
   Asn Tyr Cys Arg Val Thr Val His Asn Asn Gln Thr Thr Gly His Gly
   225 230 235 240
   Thr Lys Val Lys Gly Asn Met Ala Tyr Asp Thr His Gln Gln Ile Trp
   245 250 255
   Thr Pro Trp Ser Leu Val Asp Ala Asn Ala Trp Gly Val Trp Phe Gln
   260 265 270
   Pro Ser Asp Trp Gln Phe Ile Gln Asn Ser Met Glu Ser Leu Asn Leu
   275 280 285
   Asp Ser Leu Ser Gln Glu Leu Phe Asn Val Val Val Lys Thr Val Thr
   290 295 300
   Glu Gln Gln Gly Ala Gly Gln Asp Ala Ile Lys Val Tyr Asn Asn Asp
   305                 310                 315                 320
   Leu Thr Ala Cys Met Met Val Ala Leu Asp Ser Asn Asn Ile Leu Pro
                   325                 330                 335
   Tyr Thr Pro Ala Ala Gln Thr Ser Glu Thr Leu Gly Phe Tyr Pro Trp
               340                 345                 350
   Lys Pro Thr Ala Pro Ala Pro Tyr Arg Tyr Tyr Phe Phe Met Pro Arg
           355                 360                 365
   Gln Leu Ser Val Thr Ser Ser Asn Ser Ala Glu Gly Thr Gln Ile Thr
       370                 375                 380
   Asp Thr Ile Gly Glu Pro Gln Ala Leu Asn Ser Gln Phe Phe Thr Ile
   385                 390                 395                 400
   Glu Asn Thr Leu Pro Ile Thr Leu Leu Arg Thr Gly Asp Glu Phe Thr
                   405                 410                 415
   Thr Gly Thr Tyr Ile Phe Asn Thr Asp Pro Leu Lys Leu Thr His Thr
               420                 425                 430
   Trp Gln Thr Asn Arg His Leu Ala Cys Leu Gln Gly Ile Thr Asp Leu
           435                 440                 445
   Pro Thr Ser Asp Thr Ala Thr Ala Ser Leu Thr Ala Asn Gly Asp Arg
       450                 455                 460
   Phe Gly Ser Thr Gln Thr Gln Asn Val Asn Tyr Val Thr Glu Ala Leu
   465                 470                 475                 480
   Arg Thr Arg Pro Ala Gln Ile Gly Phe Met Gln Pro His Asp Asn Phe
                   485                 490                 495
   Glu Ala Asn Arg Gly Gly Pro Phe Lys Val Pro Val Val Pro Leu Asp
               500                 505                 510
   Ile Thr Ala Gly Glu Asp His Asp Ala Asn Gly Ala Ile Arg Phe Asn
           515                 520                 525
   Tyr Gly Lys Gln His Gly Glu Asp Trp Ala Lys Gln Gly Ala Ala Pro
       530                 535                 540
   Glu Arg Tyr Thr Trp Asp Ala Ile Asp Ser Ala Ala Gly Arg Asp Thr
   545                 550                 555                 560
   Ala Arg Cys Phe Val Gln Ser Ala Pro Ile Ser Ile Pro Pro Asn Gln
                   565                 570                 575
   Asn Gln Ile Leu Gln Arg Glu Asp Ala Ile Ala Gly Arg Thr Asn Met
   580 585 590
   His Tyr Thr Asn Val Phe Asn Ser Tyr Gly Pro Leu Ser Ala Phe Pro
   595 600 605
   His Pro Asp Pro Ile Tyr Pro Asn Gly Gln Ile Trp Asp Lys Glu Leu
   610 615 620
   Asp Leu Glu His Lys Pro Arg Leu His Val Thr Ala Pro Phe Val Cys
   625 630 635 640
   Lys Asn Asn Pro Pro Gly Gln Leu Phe Val His Leu Gly Pro Asn Leu
   645 650 655
   Thr Asp Gln Phe Asp Pro Asn Ser Thr Thr Val Ser Arg Ile Val Thr
   660 665 670
   Tyr Ser Thr Phe Tyr Trp Lys Gly Ile Leu Lys Phe Lys Ala Lys Leu
   675 680 685
   Arg Pro Asn Leu Thr Trp Asn Pro Val Tyr Gln Ala Thr Thr Asp Ser
   690 695 700
   Val Ala Asn Ser Tyr Met Asn Val Lys Lys Trp Leu Pro Ser Ala Thr
   705 710 715 720
   Gly Asn Met His Ser Asp Pro Leu Ile Cys Arg Pro Val Pro His Met
   725 730 735
   Thr Tyr
<210> 4
   <211> 5
   <212> PRT
   <213> artificial sequence
<220>
   <223> amended sequencing errors in VP1/2
<400> 4
   Gly Met Pro Pro Arg
   1 5
<210> 5
   <211> 110
   <212> DNA
   <213> artificial sequence
<220>
   <223> variation no. 1
<400> 5
   gcacaccaaa tactcctgtt gcgggcactg cagcaagcca aaacactggg gaggctggtt 60
   ccacagcctg ccaaggtgct caacggagcc caacctggtc cgagatcgag 110
<210> 6
   <211> 20
   <212> DNA
   <213> artificial sequence
<220>
   <223> variation no. 2
<400> 6
   gctggcgagg actatgatgc 20
<210> 7
   <211> 20
   <212> DNA
   <213> artificial sequence
<220>
   <223> variation no. 3
<400> 7
   acaacatggc gaaaattggg 20
<210> 8
   <211> 20
   <212> DNA
   <213> artificial sequence
<220>
   <223> variation no. 4
<400> 8
   gcgagaaaac gccatagctg 20
<210> 9
   <211> 19
   <212> DNA
   <213> artificial sequence
<220>
   <223> variation no. 5
<400> 9
   tataaaaata acataatat 19
<210> 10
   <211> 22
   <212> DNA
   <213> artificial sequence
<220>
   <223> variation no. 6
<400> 10
   aacataatat ggtattggtt aa 22
<210> 11
   <211> 20
   <212> DNA
   <213> artificial sequence
<220>
   <223> variation no. 7
<400> 11
   ctgtaaaaaa caatagaact 20
<210> 12
   <211> 78
   <212> DNA
   <213> artificial sequence
<220>
   <223> variation no. 8
<400> 12
   agatagaata taagaagatt ttgtatttta aaataaatat agttagttgg ttaatgttag 60
   atagaatata aaaagatt 78

## Description

The present invention relates to a rodent parvovirus variant capable of propagating and spreading through human tumor cells which is obtainable by serially passaging a rodent parvovirus as starting strain in semi-permissive human tumor cells. It also relates to a parvovirus variant capable of propagating and spreading through human tumor cells characterized by particular amino acid deletions and/or substitutions, e.g. a deletion of several amino acids in the C-terminus of NS1/middle exon of NS2. The present invention also relates to a pharmaceutical composition containing such parvovirus as well as its use for the treatment of cancer, preferably a glioblastoma.

Oncolytic viruses such as rodent parvoviuses represent novel tools for cancer treatment. Besides specifically killing cancer cells (oncolysis), these agents also provide danger signals prompting the immune system to eliminate virus-infected tumours. As a consequence of oncolytic events, the innate and adaptive immune systems gain access to tumour antigens, which results in cross-priming and vaccination effects. Rodent parvoviruses are single-stranded DNA viruses possessing intrinsic oncolytic activity, i.e. they preferentially replicate in and kill cancer cells of both murine and human origin.

Glioblastoma is a devastating disease with only limited treatment possibilities. Evidently, the prognosis for patients requires new therapies. The replication competent oncolytic viruses discussed above are considered promising since they are able to spread through malignant tissues and induce anti-tumor immune-responses. Among them, rodent parvoviruses appear to be excellent candidates, due to their natural oncotropism, their capacity to infect human cells, the specific toxicity for neoplastic cells, and the low pathogenicity for humans. In a rat model, H1-PV was able to cause a complete regression of established gliomas without any relaps and they proved able to target various human gliomas, thereby efficiently killing these cells irrespective of their acquired resistance towards known death inducers. Although, H1-PV proved able to infect and efficiently kill most of the human glioblastoma cell lines tested so far, the majority of these cells resisted efficient virus replication, progeny particle production and spreading. This failure to propagate and spread through human tumor tissue could have major impact for the treatment efficacy of human gliomas *in vivo*, not only because cell killing is limited to a single hit event reaching only a limited proportion of the tumor cells, but also due to the lack of an active intracellular transport of progeny virions to the cell surface.

Therefore, it is the object of the present invention to overcame this draw back of the prior art strains, i.e., to provide parvoviruses that are capable to propagate and spread through human tumor tissue.

According to the invention this is achieved by the subject matters defined in the claims. Initial experiments of the inventors have shown that progeny PV virions are shuttled from the nucleus to the cell periphery through vesicles prior to cell lysis where they are released into the medium supernatant. Moreover, this vesicular egress is associated with multiple intracellular proteins (including potential tumor antigens) which are carried as "co-cargo" to the cell surface and might contribute to the host anti-tumor immune stimulation. To mend the defects of the original H1-PV isolate (pSR19) in virus propagation and spreading in human glioblastoma, the inventors aimed to obtain propagation competent H1-PV variants through serial passaging in semi-permissive patient-derived human glioblastoma cell lines, i.e., to circumvent potential bottle-necks during the infection process (uncoating, delivery of the viral genome to the nucleus, double-strand conversion), infectious clone DNA of H1-PV (pSR-19) was transfected into different human glioblastoma cell lines (e.g. NCH149, NCH82, NCH89) and this replication competent DNA was passaged until cytopathic effects became visible. Additional viral passages (> 15) were made combining cell associated progeny virions obtained through repeated freezing and thawing in vTE pH 8.7 with medium released viruses through infection. A pool of propagation competent viruses was then analyzed after 25 passages on NCH149 cells presenting efficient viral DNA amplification, generation of progeny virions and spreading through a variety of human glioblastoma cultures. From this original pool, individual virus clones were isolated through plaque-purification on NB 324K cells, amplified again on NCH149 cells, cloned and sequenced.

The genetic analysis of all H1-PV variants has revealed a deletion of 84 nts affecting 28 amino acids of the coding region of NS1 (C-terminus) and NS2 and a single cytosine to thymidine transition at position 3913 changing His374 to Tyr in VP2. Two additional guanidine to adenosine substitutions in the VP2 region (3964 and 4108) leading to a change from Asp391 to Asn and Asp439 to Ser have only been assigned to two isolates. A few changes in the 3' non-coding regions remained of various prevalence. Apparently, the region approximately between nts 2000 to 2200 comprises a hot-spot of variability allowing H1-PV through modulation of NS1/NS2 function to adapt to the host environment. Therefore, it is possible to modify actively the host range of H1-PV, but also the other closely related rat PV strains, H3 and TVX through insertion/deletions within this region.

The newly generated H1-PV variants are able to propagate and spread through human glioblastoma cell(line)s. This allows an increased infection/destruction of already established human tumors and in consequence it is expected to give rise to a better anti-tumor immune stimulation. In agreement with the inventors' previous findings of parvovirus vesicular egress, it is reasonable to assume that the co-transport of intracellular proteins to the surface might serve to unmask tumor cells for a host immune response. This co-cargo effect is not present in absence of progeny virion formation using the original H1-PV isolate (pSR-19).

### Summary of the present Invention

With the adaptation process (passaging) in semi-permissive cells as outlined in the examples an H1-PV variant proficient for propagation in many other human glioblastoma cell lines could be generated. The outlined procedure can be expanded to other human tumor cell(line)s including tumor stem cells.

Adaptation of the rat H1-PV on human glioblastoma cells led to a deletion in the C-terminus of H1 NS1/middle exon of NS2. This region represents a hot spot area for variation determining H1-PV host range and with additional adapatations/analyses of other so far uncharacterized rodent PV isolates additional variations can be found in this area.

Besides generating an H1-PV variant that is potentially able to spread from cell to cell in human glioblastoma tumor tissue and therefore has a higher potential for tumor cell killing (as indicated by the fitness assays), progeny particles of this isolate are actively transported to the cell periphery through vesicles. Besides newly synthesized virions, multiple cellular proteins being associated with such vesicles were found after infection which were not present in non-infected human glioblastoma cells. This co-cargo could serve to unmask tumor cells to make them available for the host immune system, thereby contributing for a virus-induced anti-tumor immune response.

### Brief description of the drawings

Figure 1:
   (Top) Schematical presentation of the adaptation approach Rat parvovirus H1-PV readily propagates in transformed rat cells such as rat glioma RG-2 (left), but is limited to produce and spread in human glioblastoma cell lines. To overcome these limitations, serial passages were performed in semi-permissive human glioblastoma allowing mutations and amplification of permissive H1-PV variants to occur. Propagation competent variants were obtained/analyzed after 20 consecutive passages in NCH149 cells.
   (B, C) Determination of viral DNA amplification in NCH149 NCH149 cells were infected with 10 pfu/cell H1-PV and the presence of replication intermediates was measured by Southern blotting after indicated times post infection.
   **(B)** Infection with rat H1-PV (pSR19).
   **(C)** Primary and secondary DNA amplification with human glioma adapted H1-PV, determination of gelsolin functioning for vesicular egress and spreading. Inf 1, primary infection, Inf 2, supernatants from Inf 1 were used to infect naive NCH149 cells. The generation of viral replication intermediates was measured by Southern blotting. αCon, control serum; αGln, neutralizing antiserum raised against gelsolin; dRF, dimeric replication intermediate; mRF, monomeric replication intermediate; ssDNA, progeny virus particles = single-stranded virus genomes.

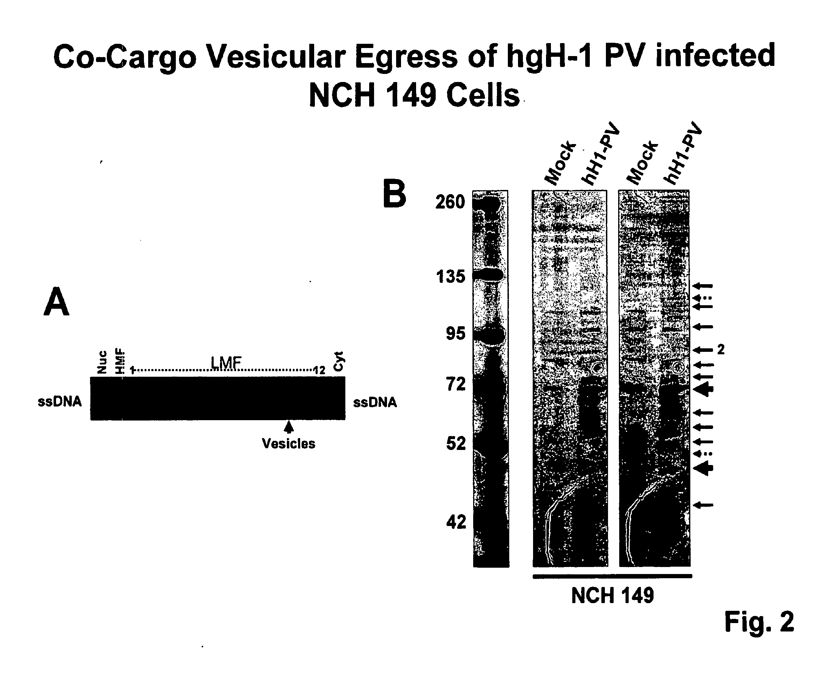

Figure 2: Determination of H1-PV vesicular egress and identification of "co-cargo"
   **(A)** NCH149 cells were infected with 10 pfu/cell human glioma adapted H1-PV and fractionated for nuclei (Nuc), heavy mitochondrial fraction (HMF), and light mitochondrial fraction (LMF), containing cellular vesicles. To determine the presence of progeny virions in the vesicular fraction, LMF components were further separated by iodexanol gradients and the presence of virion DNA was measured by Southern blotting as compared to the presence of vesicular markers (determined by Western blotting).
   **(B)** Determination of vesicular proteins in hgH1-PV and mock infected NCH149 cells. Vesicles were purified as described under (A) and analyzed for their protein content by SDS-PAGE and silver staining. Proteins overrepresented in hgH1-PV infected cellular vesicles are indicated by arrows.
Figure 3:
   (A) Determination of viral protein expression after infection of NCH149 cells with rat (pSR19) versus human glioma adapted H1-PV
      NCH149 cells were infected with 10 pfu/cell and harvested at indicated times post infection. Accumulation of viral proteins were determined by Western blotting. Encircles (red) the large heterogeneity of NS1 produced after hgH1-PV infection and the larger accumulation of NS2 and NS3 (blue).
   (B) RG-2 and NCH149 cells were infected with the indicated virus preparations at a multiplicity of 10, and metabolically labelled with ³²P-orthophosphate at 24 h p.i. for four hours Cells were harvested, NS1 purified by immunoprecipitations and SDS-PAGE and the tryptic phosphopeptide pattern was determined by two-dimensional electrophoresis/chromatography. The phosphopeptide pattern of NS1 produced by rat H1-PV (pSR-19) in rat glioma (left), NCH149 (middle), as well as hgH1-PV in NCH149 cells (right) are shown. Encircled: phosphorylations known to drive viral DNA amplification.
Figure 4:
   (A) Multiplication of input virus after infection of various target cell lines
      Indicated cell lines were infected at a multiplicity 10, washed and harvested 4 (Input virus) 24 and 48 h post infection. Cell and medium associated virus was determined by plaque assay. The average amplification 24 and 48 h post infection is expressed over input (4 h) virus titer.
   (B) Virus fitness assay
      Target cell lines were seeded in 24 well dishes, infected at indicated multiplicities with the appropriate virus preparations, and living cells were fixed and stained with crystal violet 6 days post infection.
Figure 5:
   Top: Genome organization of H1-PV with indication of variation found after adaptation in human glioma
   Middle section: PCR-fragments generated from plaque purified virus preparations
   Bottom: Genetic determination of plaque purified hgH1-PV variants
   Cloned PCR fragments are indicated by black lines, sequenced material is indicated by dotted squares and the presence of the individual variations are indicated in green.
Figure 6: Detailed characterization of all genetic variations found by sequencing of individual plaques
Figure 7: Virus fitness assay of virus stocks derived from individual plaque isolates

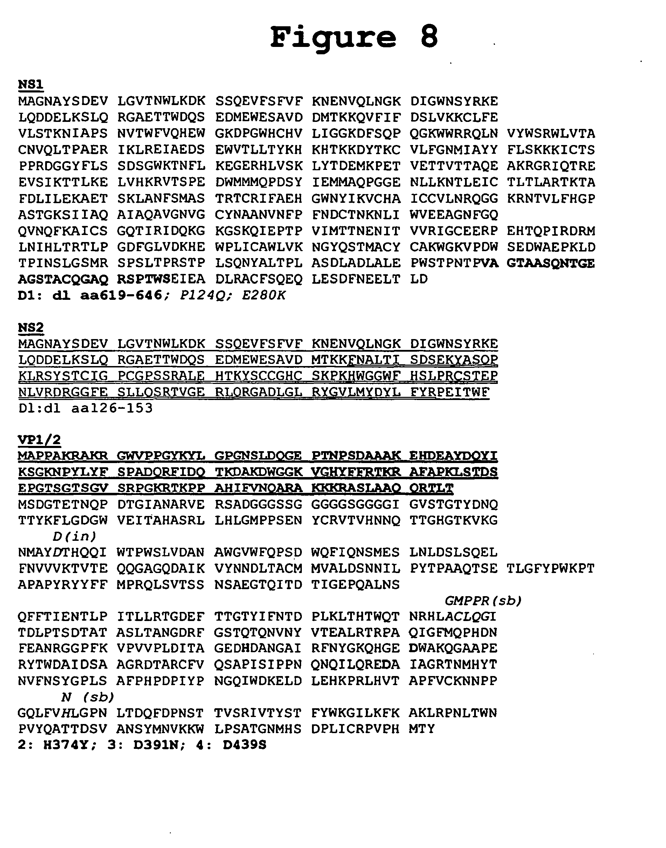

Figure 8: Protein sequences corresponding to the H1-PV nucleotide sequence published under NC 001358

The three exons of NS2p as deduced from the transcription pattern of Qiu et al., (2006) are underlined (common N-terminus with NS1 = exon1)- (middle exon: double underlining)-yellow (C-terminal exon [unique for each NS2 isoform). Amended sequencing errors are italicized (these differences to the published sequence were found in all isolates as well). Variations created through passaging in NCH149 cells are bold. VP1-specific sequences are bold and underlined.

### Detailed Description of the present Invention

The present invention provides a parvovirus variant capable of propagating and spreading through human tumor cells characterized in that it comprises a deletion of 28 amino acids from position 619 to 646 (Rhode and Paradiso, 1983) in the C-terminus of NS1/position 126 to 153 in the middle exon of NS2 (Rhode and Paradiso, 1983; Qiu et al., 2006), and an H374Y substitution in VP1/2.

The present invention also provides a parvovirus variant capable of propagating and spreading through human tumor cells characterized in that it comprises the following amino acid substitution(s) in VP1/2:
(a) D391N; and/or
(b) D439S.

The term "propagating" as used herein means that the progeny virions of the parvovirus variant show amplification significantly over input levels (i.e. > 5 fold).

The term "spreading through" as used herein means that the progeny virions of the parvovirus variant are capable not only of killing primarily infected cell populations, but are able to cause secondary infections of naive cells with particles produced during the first round of infection.

The term "semi-permissive" as used herein for characterizing the host cells means that the infection with the virus results in incomplete viral replication within the particular type of host cells but are characterized by viral DNA amplification and eventually low yields of progeny virions. The latter do not necessarily have to be infectious in the beginning of the procedure.

The expression "parvovirus" comprises any parvovirus, particularly a rodent parvovirus, such as minute virus of mice (MVM) and H-1 virus as well as related viruses or vectors based on such viruses or derivatives. The person skilled in the art can arrive at the parvovirus variant according to the present invention by subjecting a starting strain to serial passaging as outlined in the Examples, below, or by introducing a deletion resulting in the desired modification of the biological properties, i.e., capability of the parvovirus variant to propagate and spread through, thereby killing, human tumor cells starting from the known nucleic acid sequence and amino acid sequences of the non-structural proteins of parvoviruses, e.g., from parvovirus H-1 (Rhode and Paradiso, 1983; Qiu et al., 2006). The person skilled in the art can also easily assay whether a particular variant exhibits the desired biological properties recited by using the assays explained in the examples, below.

Preferably, the parvovirus variant of the invention is derived from parvovirus H-1 (H-1PV) or a related rodent parvovirus. Examples of preferred related rodent parvovirus are LuIII, Mouse minute virus (MMV), Mouse parvovirus (MPV), Rat minute virus (RMV), Rat parvovirus (RPV) or Rat virus (RV).

In principle, any tumor can be cured using a parvovirus variant of the present invention with gliomas being preferred.

A particular preferred glioma is a glioblastoma.

A preferred human semi-permissive tumor cell line is NCH149.

As regards the procedure of serial passaging, the person skilled in the art can easily determine how many passages are sufficient for arriving at a parvovirus of the present invention. In general, however, this method of the invention comprises at least 10 passages, preferably at least 20 to 25 passages, or more. Preferably, passaging is on NCH149 cells.

In order to ensure the purity and stability of the virus variants the individuals clones can be plaque-purified after passaging, preferably on NB 324K cells.

The present invention also provides a method of generating a parvovirus variant capable of propagating and spreading through human tumor cells said method comprising serially passaging a rodent parvovirus as starting strain in semi-permissive human tumor cells as described above and in the examples, below.

A further subject matter of the present invention relates to a nucleic acid, particularly a DNA, which codes for an above parvovirus variant.

A DNA according to the invention can be present in a vector and expression vector, respectively. A person skilled in the art is familiar with examples thereof. In the case of an expression vector for E. coli these are e.g. pGEMEX, pUC derivatives, pGEX-2T, pET3b, T7 based expression vectors and pQE-8. For the expression in yeast, e.g. pY100 and Ycpad1 have to be mentioned while e.g. pKCR, pEFBOS, cDM8, pMSCND, and pCEV4 have to be indicated for the expression in animal cells. The baculovirus expression vector pAcSGHisNT-A is especially suitable for the expression in insect cells.

In a preferred embodiment, the vector containing the DNA according to the invention is a virus, e.g. an adenovirus, vaccinia virus, an AAV virus or a parvovirus, such as MVM or H-1, a parvovirus being preferred. The vector may also be a retrovirus, such as MoMULV, MoMuLV, HaMuSV, MuMTV, RSV or GaLV.

For constructing expression vectors which contain the DNA according to the invention, it is possible to use general methods known in the art. These methods include e.g. in vitro recombination techniques, synthetic methods and in vivo recombination methods.

Furthermore, the present invention relates to host cells which contain the above described variants or vectors. These host cells include human 293(T), NBK, rat RG2 cells, NCH149, NCH82, or U87 cells. Methods of transforming these host cells, of phenotypically selecting transformants and of expressing the DNA according to the invention by using the above described vectors are known in the art.

Thus, the present invention also provides a method of producing the parvovirus variant of the invention, comprising the culturing of a host cell of the invention under suitable conditions and harvesting the parvovirus variant from the cells or the medium.

Moreover, the present invention relates to antibodies which specifically recognize an above describe parvovirus variant, i.e. the polypeptide region of the parvovirus variant where the deletion is located characterizing the rodent parvovirus variant in that it is capable of propagating and spreading through human gliomas. The antibodies can be monoclonal, polyclonal or synthetic antibodies or fragments thereof, e.g. Fab, Fv or scFV fragments. Preferably monoclonal antibodies are concerned. For the production it is favorable to immunize animals - particularly rabbits or chickens for a polyclonal antibody and mice for a monoclonal antibody - with an above parvovirus variant or with fragments thereof. Further boosters of the animals can be affected with the same parvovirus variant or with fragments thereof. The polyclonal antibody can then be obtained from the animal serum and egg yolk, respectively. The monoclonal antibody can be obtained according to standard methods, reference being made particularly to the method by Köhler and Milstein (Nature 256 (1975), 495) and Galfre (Meth. Enzymol. 73 (1981), 3). In this case, mouse myeloma cells are fused with spleen cells originating from the immunized animals. Antibodies according to the invention can be used in many ways, e.g. for the immunoprecipitation of the above described parvovirus variants or for the isolation thereof. The antibodies can be bound in immunoassays in liquid phase or to a solid carrier. In this connection, the antibodies can be labeled in various ways. The person skilled in the art is familiar with suitable markers and labeling methods. Examples of immunoassays are ELISA and RIA.

A kit is also provided for the application of the present invention. This kit comprises the following:
(a) a parvovirus variant according to the invention;
(b) a DNA according to the invention, e.g. an expression vector, particularly a parvovirus;
(c) an antibody according to the invention; and/or, optionally,
(d) conventional auxiliary agents, such as solvents, buffers, carriers, markers and controls.

Of component (a) to (d) one or more representatives can be present each.

The present invention also provides a pharmaceutical composition containing a parvovirus variant of the invention, vector or a cell producing said parvovirus variant ("parvotherapeutic agent" or "parvotherapeutic composition"), e.g. human 293(T), NBK or rat RG2 cells.

For administration, the parvotherapeutic agent can be combined with suitable pharmaceutical carriers. Suitable pharmaceutical carriers of a type well known in the art and readily commercially available, include phosphate buffered saline (PBS) solutions, water, emulsions such as oil/water emulsions, wetting agents of various types, sterile solutions, etc. Such carriers can be formulated with the parvotherapeutic agent(s) by conventional formulating methods for administration to the subject at a suitable dose.

Additional pharmaceutically compatible carriers can include gels, biosorbable matrix materials, implantation elements containing the therapeutic agent, or any other suitable vehicle, delivery or dispensing means or material(s).

Administration of the parvotherapeutic pharmaceutical compositions to a patient, e.g. a brain tumor patient, may be effected in any of numerous suitable ways, e.g., by intravenous, intraperetoneal, subcutaneous, intramuscular, topical, intradermal, intracranial, and intratumoral administration. The route of administration, of course, depends on the nature of the disease and the specific therapeutic agent(s) contained in the pharmaceutical composition.

If such parvotherapeutic agent(s) comprise infectious virus particles with the ability to penetrate through the blood-brain barrier, treatment can be performed or at least initiated by intravenous injection of the viral therapeutic agent, e.g., a H1-PV variant.

Since long-term intravenous treatment is susceptible to becoming inefficient as a result of the formation of neutralizing antibodies to the viral therapeutic agent, different modes of administration can be adopted after an initial regimen of intravenous viral administration, or such different administration techniques, e.g., intracranial or intratumoral virus administration, can be alternatively used throughout the entire course of parvoviral treatment.

As another specific administration technique, the parvotherapeutic agent (virus, vector and/or cell agent) can be administered to the patient from a source implanted in the patient. For example, a catheter, e.g., of silicone or other biocompatible material, can be connected to a small subcutaneous reservoir (Rickham reservoir) installed in the patient during tumor removal or by a separate procedure, to permit the parvotherapeutic composition to be injected locally at various times without further surgical intervention. The parvovirus variant or derived vectors can also be injected, e.g., into a tumor, by stereotactic surgical techniques or by neuronavigation targeting techniques.

Administration of the parvoviral agents or compositions can also be performed by continuous infusion of viral particles or fluids containing viral particles through implanted catheters at low flow rates using suitable pump systems, e.g:, peristaltic infusion pumps or convection enhanced delivery (CED) pumps.

A yet another method of administration of the parvotherapeutic composition is from an implanted device constructed and arranged to dispense the parvotherapeutic agent to the desired locus, e.g., tumor. For example, wafers can be employed that have been impregnated with the parvotherapeutic composition, e.g., a parvovirus H-1 variant, wherein the wafer is attached to the edges of the resection cavity at the conclusion of surgical tumor removal. Multiple wafers can be employed in such therapeutic intervention.

Cells that actively produce the parvotherapeutic agent, e.g., a parvovirus H-1 variant, or H-1 variant vectors, can be injected into the desired tumor, or into a tumoral cavity after tumor removal.

Combinations of two or more of the above-described administration modes can be employed in any suitable manner, e.g., concurrently, contemporaneously, or sequentially.

The dosage regimen of the parvotherapeutic agent is readily determinable within the skill of the art, by the attending physician based on patient data, observations and other clinical factors, including for example the patient's size, body surface area, age, sex, the particular virus, vector, cell, etc. to be administered, the time and route of administration, the tumor type and characteristics, general health of the patient, and other drugs or therapies to which the patient is being subjected.

Accordingly, the present invention also relates to the use of a parvovirus variant according to the present invention, a cell producing said parvovirus variant, a DNA, an expression vector or antibody of the present invention for the preparation of a pharmaceutical composition for the treatment of cancer. Brain tumors (preferably a glioma, medulloblastoma, meningioma or glioblastoma) are expected to be particularly amenable to treatment with an agent of the present invention.

The below examples explain the invention in more detail.

### Example 1

### Adaptation of rat H1-PV (pSR19) to human glioma

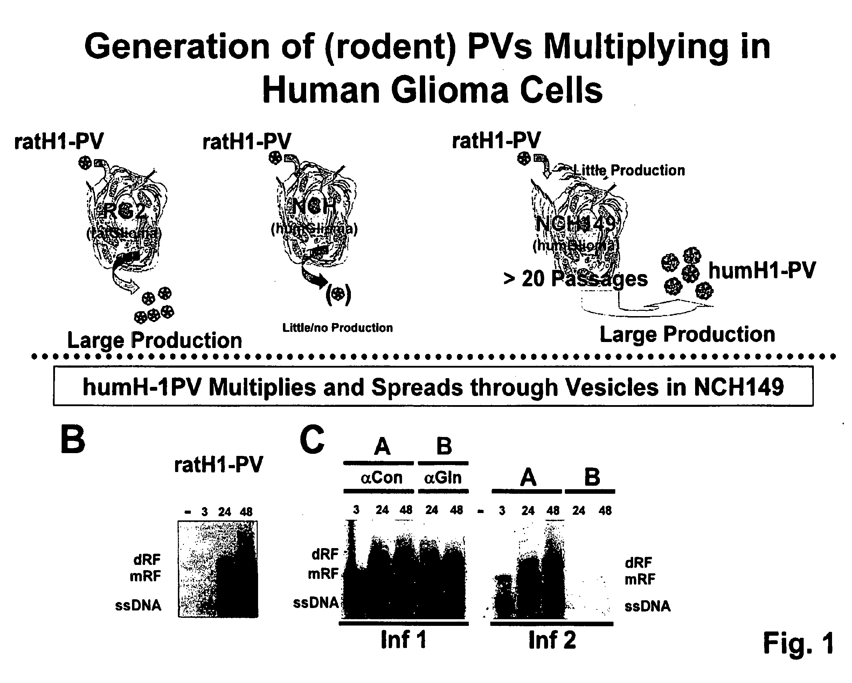

Infection of many human glioblastoma cell lines with H1-PV derived from pSR19 isolate (Accession No: NC_001358) led to the expression of viral proteins and in consequence cell killing irrespective of their resistance towards drug-induced apoptosis. However, monitoring viral DNA amplification and progeny particle production, with exception of NCH89, the majority of the tested human glioblastoma cell lines only allowed little DNA amplification and virtually no increase in progeny production (Herrero y Calle et al., 2004; DiPiazza et al., 2007). To mend this defect, it was aimed to obtain (a) propagation competent H1-PV variant(s) through serial passaging of pSR19 in human glioblastoma cells, which were competent for DNA amplification although progeny virion production was hampered. The infectious clone DNA of H1-PV, pSR19 was transfected into NCH149 cells, a cell line that was shown to allow low levels of DNA amplification by Southern blotting (Fig. 1B). Parvoviruses are dependent on (rapidly) growing cells, since conversion of the single-stranded genome to a double-stranded transcription template requires S-phase. Therefore, the initial inoculum (tranfected cells) was first amplified by dilutions of 1:4 after confluency on the surface. After reaching 4x10⁸ cells, ¾ of the cells were harvested, progeny virions were released by repeated freezing and thawing into the supernatant, and a combination of naive and transfected cells was re-infected with these virions until confluency or CPE was obtained. This procedure was continued until passage 20-25.

### Example 2

### Analyses of human glioma adapted H1-PV

After more than 20 consecutive passages the inventors obtained a virus stock competent for viral DNA amplification, progeny virion production and spreading in NCH149 cultures. The properties of this virus pool were analyzed. As shown in Fig. 1C by Southern blotting, this virus pool was characterized by strong DNA amplification after infection of NCH149 cells, producing monomeric and dimeric replication intermediates as well as single-stranded virion DNA. Besides reflecting the production of genomic viral DNA, this latter form indicates the formation of progeny virions, since the generation of single-stranded DNA is associated with the packaging process. Moreover, when supernatants of infected cells were collected (Inf 1) to infect naive NCH149 cells (Inf 2) similar DNA amplification and generation of single-stranded virion DNA was observed, demonstrating production and release of large numbers of infectious progeny virions in NCH149 cells. Indeed, release of progeny virions into the medium supernatants is dependent on the activity of cellular gelsolin, since application of neutralizing gelsolin antibodies inhibited this process. This dependency on gelsolin for the release of progeny virions indicates the involvement of an active vesicular transport to the cell periphery of progeny virions (Bar et al., 2008).

As shown in Fig. 2A by Southern blotting of the single-stranded progeny virion DNA after biochemical fractionations progeny H1-PV variant particles are found being strongly associated with vesicular fractions of infected cells. Moreover, by comparing vesicles from non-infected versus infected NCH149 cells analyzing the protein content by silver-staining a number of proteins being associated with virion-containing vesicular fractions, while being absent in non-infected cells was identified. The nature of these proteins is currently determined by MS/MS analyses.

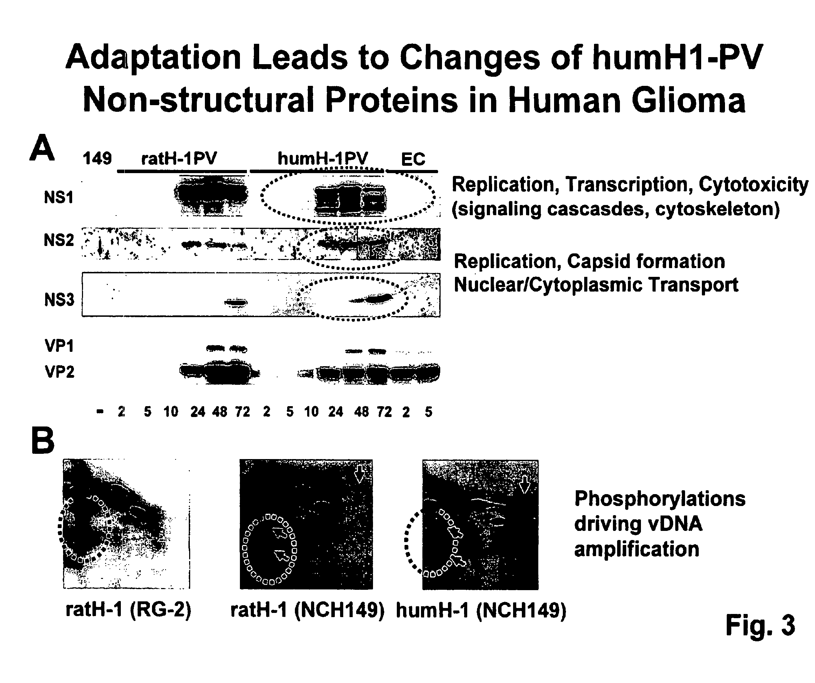

Then, the nature of the virus modification(s) causing the permissive phenotype in NCH149 cells was identified. Original H1-PV (pSR19) was produced in rat RG-2 glioma, whereas the adapted H1-PV variant (hgH1-PV) was generated in NCH149. Both virus stocks were titrated by plaque assays in permissive NB324K cells. Infections were performed at a multiplicity of 10 in NCH149 cells and the production of viral proteins was determined in a time-course experiment by Western blotting. As shown in Fig. 3A, remarkable differences in the expression levels of the small non-structural proteins NS2 and NS3 were observed, while NS1 from hgH1-PV proved to be by far more heterogenous than NS1 from pSR19. NS1 is a multifunctional phosphoprotein, which is required for many processes during virus amplifications and is regulated for its functioning by differential phosphorylations. Indeed, as shown in Fig. 3B, when the phosphorylation patterns of NS1 obtained from pSR19 after infections of rat RG-2 cells and human NCH149 cells was analyzed the lack of two characteristic peptides after infection of NCH149 (encircled) was observed. In contrast, hgH1-PV was able to generate these phosphopeptides (and an additional peptide marked with an arrow), accounting for the differences in the migration pattern observed by Western blotting. Moreover, detailed functional analyses of the MVM NS1 phosphorylation (pattern), which closely resembles the one observed for pSR19 in RG-2/hgH1-PV in NCH149 suggests that the two encircled peptides are important phosphorylations involved in the replicative functions of the NS1 protein.

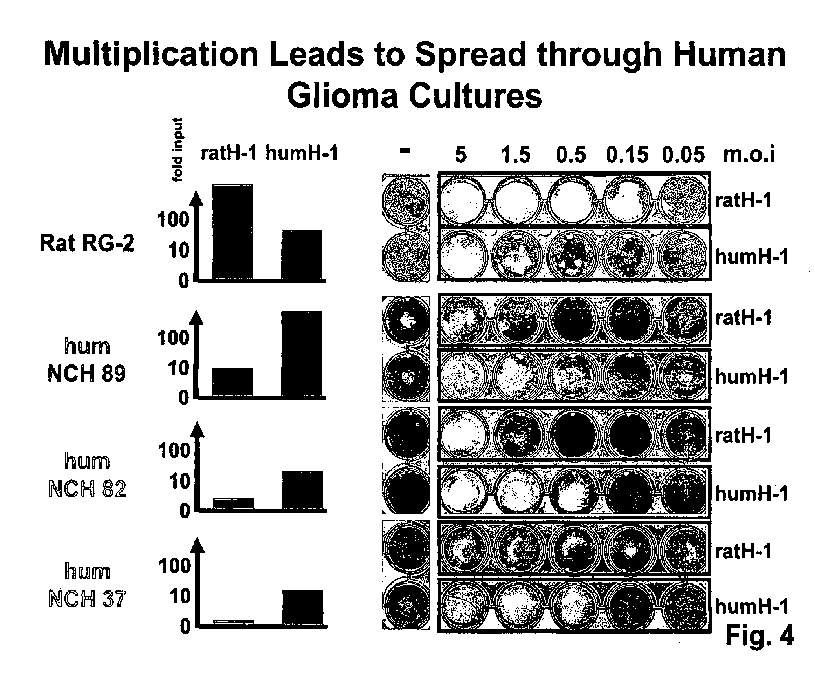

Finally, the adapted hgH1-PV stock was tested for its fitness to propagate and spread through a variety of (human) glioblastoma cultures in comparison to the pSR19 isolate. As shown in Fig. 4. in addition to NCH149 cells, all other human glioblastoma cell lines tested so far proved to be proficient to amplify hgH1-PV (but not pSR19) progeny virions at least ten fold above input levels and proved superior to pSR19 to spread through the cultures.

### Example 3

### Genetic characterization of human glioma adapted H1-PV

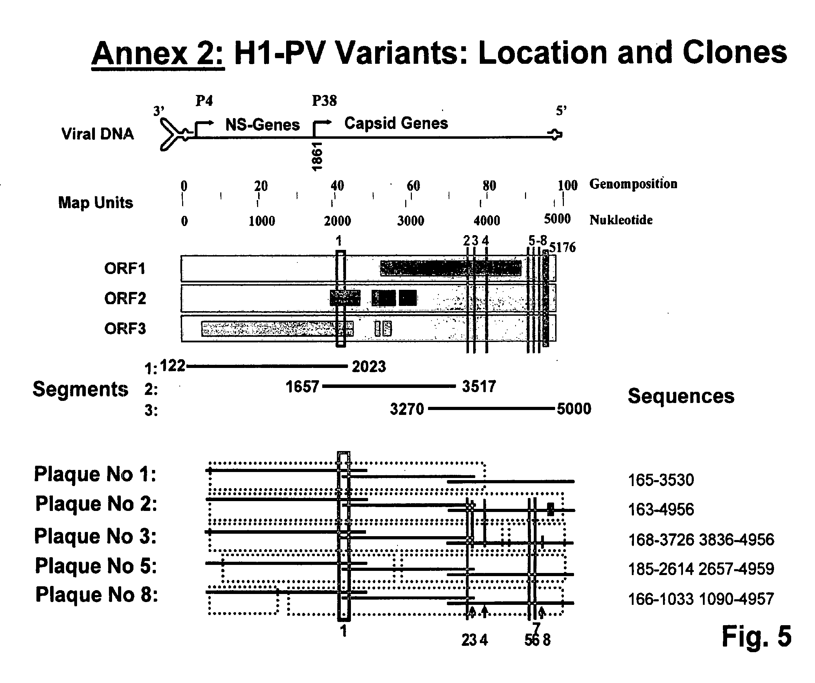

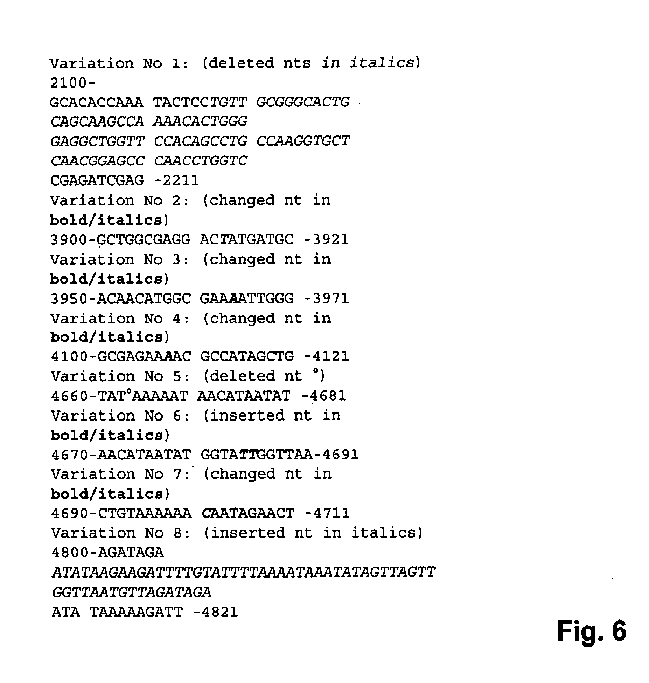

To determine genetic variations hgH1-PV acquired during passaging in NCH149 as compared to the original inoculum (pSR19), endpoint titrations were performed to plaque purify individual virus variants and virion DNA was isolated. As shown in Fig. 5, DNA of five individual plaques was obtained, the whole coding region was amplified by PCR in three steps and the overlapping fragments were cloned into pCR2.1. Genetic variations were determined by sequencing and compared to the published sequence of pSR19 (Accession No NC_001358). Mutations as compared to the pSR19 sequence found to be present on the individual plaque isolates are indicated, numbered and positioned in the H1-map. The detailed sequence alterations are shown in Fig. 6.

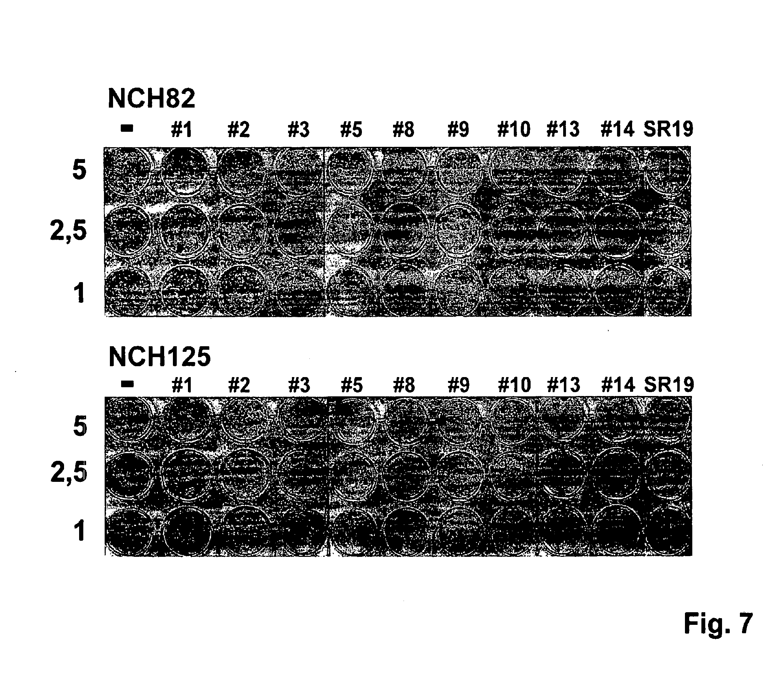

To further characterize the properties of individual clones, virus stock of individual plaque isolates was generated and their fitness to spread and kill human glioblastoma cell cultures in comparison to pSR19 generated in rat RG-2 cells was tested. As shown in Fig. 7, all isolates harbouring a deletion mutant in the C-terminal part of NS1/middle exon of NS2 surpassed pSR19 in their fitness to spread and kill human glioblastoma cells, confirming the initial findings with the pooled variants and demonstrating that the deletion mutant is responsible for the observed host range switch.

### List of References

Rhode, S.L. III and Paradiso, P.R. (1983. Parvovirus genome: nucleotide sequence of H-1 and mapping of its genes by hybrid-arrested translation. J. Virol. 45, 173-184.
Qiu, J., Yoto, Y., Tullis, G., and Pintel, D.J. (2006). Parvovirus processing strategies. pp.253-274. In "Parvoviruses" Eds. Kerr, J.R., Cotmore, S.F., Bloom, M.E., Linden, R.M., and Parrish, C.R. Hodder Arnold, London.

## Claims

1. A rodent parvovirus variant capable of propagating and spreading through human tumor cells, **characterized in that** the parvovirus variant comprises (a) a deletion of 28 amino acids from position 619 to 646 in the C-terminus of NS1/from position 126 to 153 in the middle exon of NS2 and (b) the amino acid substitution in VP1/2 H374Y.

2. The parovirus variant of claim 1, further comprising the amino acid substitutions D391N and/or D439S.

3. The parvovirus variant of claim 1 or 2, wherein the starting strain is a rat parvovirus.

4. The parvovirus variant of claim 3 which is rat parvovirus H-1.

5. The parvovirus variant of any one of claims 1 to 4, wherein the tumor cells are glioma cells.

6. The parvovorius variant of claim 5, wherein the glioma is a glioblastoma.

7. A DNA, coding for the parvovirus variant according to any one of claims 1 to 6.

8. An expression vector, comprising the DNA according to claim 7.

9. A host cell containing the parvovirus variant of any one of claims 1 to 6 or the expression vector according to claim 8.

10. An antibody, directed against the NS1 protein of a parvovirus variant according to any one of claims 1 to 6 further **characterized in that** it does only bind to the variant protein having the deletion but not to the wild type protein.

11. Kit comprising:
(a) a parvovirus variant of any one of claims 1 to 6;
(b) a DNA according to claim 7;
(c) an antibody according to claim 10; and/or, optionally,
(d) conventional auxiliary agents, such as solvents, buffers, carriers, markers and controls,
wherein of components (a) to (d) one or more representatives can be present each.

12. A pharmaceutical composition containing (a) a parvovirus according to any one of claims 1 to 6, the DNA of claim 7, the expression vector of claim 8, the host cell of claim 9 and (b) a pharmaceutically acceptable carrier.

13. Use of a parvovirus according to any one of claims 1 to 6, the DNA of claim 7, the expression vector of claim 8, the host cell of claim 9 for the preparation of a pharmaceutical composition for the treatment of cancer.

14. The parvovirus according to any one of claims 1 to 6, the DNA of claim 7, the expression vector of claim 8, the host cell of claim 9 for use in a method of treating cancer.

15. The use of claim 13 or 14, wherein said cancer is a brain tumor.

16. The use of claim 15, wherein said brain tumor is a glioma, medulloblastoma, meningioma or glioblastoma.

## Patentansprüche

1. Nagetier-Parvovirusvariante, die sich in menschlichen Tumorzellen vermehren und ausbreiten kann, **dadurch gekennzeichnet, dass** die Parvovirusvariante (a) von der Position 619 bis 646 im C-Terminus von NS1 / von der Position 126 bis 153 im mittleren Exon von NS2 eine Deletion von 28 Aminosäuren und (b) die Aminosäuresubstitution H374Y in VP1/2 aufweist.

2. Parvovirusvariante nach Anspruch 1, die weiterhin die Aminosäuresubstitutionen D391N und/oder D439S aufweist.

3. Parvovirusvariante nach Anspruch 1 oder 2, wobei der Ausgangsstamm ein Ratten-Parvovirus ist.

4. Parvovirusvariante nach Anspruch 3, bei der es sich um das Ratten-Parvovirus H-1 handelt.

5. Parvovirusvariante nach einem der Ansprüche 1 bis 4, wobei die Tumorzellen Gliomzellen sind.

6. Parvovirusvariante nach Anspruch 5, wobei das Gliom ein Glioblastom ist.

7. DNA kodierend für die Parvovirusvariante nach einem der Ansprüche 1 bis 6.

8. Expressionsvektor, umfassend die DNA nach Anspruch 7.

9. Wirtszelle, die die Parvovirusvariante nach einem der Ansprüche 1 bis 6 oder den Expressionsvektor nach Anspruch 8 enthält.

10. Antikörper, der gegen das NS1-Protein einer Parvovirusvariante nach einem der Ansprüche 1 bis 6 gerichtet ist, weiterhin **dadurch gekennzeichnet, dass** er nur die Proteinvariante mit der Deletion, aber nicht das Wildtypprotein bindet.

11. Kit umfassend:
(a) Parvovirusvariante nach einem der Ansprüche 1 bis 6;
(b) DNA nach Anspruch 7;
(c) Antikörper nach Anspruch 10; und/oder optional,
(d) herkömmliche Hilfsmittel, wie beispielsweise Lösungsmittel, Puffer, Trägerstoffe, Marker und Kontrollen,
wobei von den Bestandteilen (a) bis (d) ein oder mehrere jeweils vorhanden sein können.

12. Pharmazeutische Zusammensetzung enthaltend (a) ein Parvovirus nach einem der Ansprüche 1 bis 6, die DNA nach Anspruch 7, den Expressionsvektor nach Anspruch 8, die Wirtszelle nach Anspruch 9 und (b) einen pharmazeutisch verträglichen Trägerstoff.

13. Verwendung eines Parvovirus nach einem der Ansprüche 1 bis 6, der DNA nach Anspruch 7, dem Expressionsvektor nach Anspruch 8, der Wirtszelle nach Anspruch 9 zur Herstellung einer pharmazeutischen Zusammensetzung zur Behandlung von Krebs.

14. Parvovirus nach einem der Ansprüche 1 bis 6, der DNA nach Anspruch 7, dem Expressionsvektor nach Anspruch 8, der Wirtszelle nach Anspruch 9 zur Verwendung in einem Verfahren zur Behandlung von Krebs.

15. Verwendung nach Anspruch 13 oder 14, wobei der Krebs ein Gehirntumor ist.

16. Verwendung nach Anspruch 15, wobei der Gehirntumor ein Gliom, Medulloblastom, Meningiom oder Glioblastom ist.

## Revendications

1. Variante de parvovirus de rongeurs capable de se propager et de se répandre dans les cellules tumorales humaines, **caractérisée par le fait que** la variante de parvovirus comprend (a) une délétion de 28 acides aminés de la position 619 à 646 dans l'extrémité C-terminale de NS1/de la position 126 à 153 dans l'exon de NS2 et (2) la substitution d'acides aminés dans VP1/2 H374Y.

2. Variante de parvovirus selon la revendication 1, comprenant par ailleurs les substitutions d'acides aminés D391 et/ou D439S.

3. Variante de parvovirus selon la revendication 1 ou 2, dans laquelle la lignée de départ est un parvovirus de rat.

4. Variante de parvovirus selon la revendication 3, qui est un parvovirus de rat H-1.

5. Variante de parvovirus selon l'une quelconque des revendications 1 à 4, dans laquelle les cellules tumorales sont des cellules de gliome.

6. Variante de parvovirus selon la revendication 5, dans laquelle le gliome est un glioblastome.

7. ADN, codifiant la variante de parvovirus selon l'une quelconque des revendications 1 à 6.

8. Vecteur d'expression, comprenant l'ADN selon la revendication 7.

9. Cellule hôte contenant la variante de parvovirus selon l'une quelconque des revendications 1 à 6 ou le vecteur d'expression selon la revendication 8.

10. Anticorps, dirigé contre la protéine NS1 d'une variante de parvovirus selon l'une quelconque des revendications 1 à 6, **caractérisé par** ailleurs par le fait qu'il ne se lie qu'à la protéine modifiée présentant la délétion, mais pas la protéine de type sauvage.

11. Kit comprenant:
(a) une variante de parvovirus de l'une quelconque des revendications 1 à 6;
(b) un ADN selon la revendication 7;
(c) un anticorps selon la revendication 10; et/ou, optionnellement,
(d) des agents auxiliaires conventionnels, tels que solvants, tampons, porteurs, marqueurs et témoins,
dans lequel, parmi les composants (a) à (d), un ou plusieurs représentants peuvent chaque fois être présents.

12. Composition pharmaceutique contenant (a) un parvovirus selon l'une quelconque des revendications 1 à 6, l'ADN selon la revendication 7, le vecteur d'expression selon la revendication 8, la cellule hôte selon la revendication 9, et (b) un porteur acceptable pharmaceutiquement.

13. Utilisation d'un parvovirus selon l'une quelconque des revendications 1 à 6, de l'ADN selon la revendication 7, du vecteur d'expression selon la revendication 8, de la cellule hôte selon la revendication 9 pour la préparation d'une composition pharmaceutique pour le traitement de cancer.

14. Le parvovirus selon l'une quelconque des revendications 1 à 6, l'ADN selon la revendication 7, le vecteur d'expression selon la revendication 8, la cellule hôte selon la revendication 9 destinés à être utilisés dans un procédé de traitement du cancer.

15. Utilisation selon la revendication 13 ou 14, dans laquelle ledit cancer est une tumeur du cerveau.

16. Utilisation selon la revendication 15, dans laquelle ledit tumeur du cerveau est un gliome, un medulloblastome, un méningiome ou un glioblastome.
